# EUROPÄISCHE PATENTANMELDUNG

(11) **EP 3 708 558 A1**
(43) Veröffentlichungstag der Anmeldung: **16.09.2020**
(21) Anmeldenummer: 19163321.3
(22) Anmeldetag: 15.03.2019
(51) Int. Cl.: C07C 5/48, C07C 2/82, C07C 7/09, C07C 7/148, C07C 7/163, C07C 7/167, C07C 11/04

(54) **VERFAHREN UND ANLAGE ZUR HERSTELLUNG EINES ODER MEHRERER OLEFINE**

(71) Anmelder: Linde GmbH, 82049 Pullach (DE)
(72) Erfinder: WÖHL, Anina, 81379 München (DE); SCHUBERT, Martin, 81375 München (DE); ZELLHUBER, Mathieu, 82152 Martinsried (DE); SCHULTE, Sonja, 82515 Wolfratshausen (DE); MEISWINKEL, Andreas, 83253 Rimsting (DE); SCHÖDEL, Nicole, 81477 München (DE)
(74) Vertreter: m patent group

(57) **Zusammenfassung**

Es wird ein Verfahren (100) zur Herstellung eines oder mehrerer Olefine vorgeschlagen, bei dem ein Reaktionseinsatz gebildet wird, der Sauerstoff und ein oder mehrere Paraffine enthält, und bei dem ein Teil des Sauerstoffs in dem Reaktionseinsatz mit einem Teil des oder der Paraffine unter Erhalt eines Prozessgases durch ein oxidatives Verfahren zu dem oder den Olefinen umgesetzt wird, wobei das Prozessgas zumindest den nicht umgesetzten Teil des oder der Paraffine und des Sauerstoffs, das oder die Olefine, ein oder mehrere Acetylene, Kohlendioxid und Wasser enthält. Das das Verfahren umfasst, dass das Prozessgas oder ein Gasgemisch, das unter Verwendung zumindest eines Teils des Prozessgases gebildet wird, teilweise oder vollständig einer zumindest teilweisen Entfernung (4) des Sauerstoffs und des oder der Acetylene unterworfen wird, wobei die zumindest teilweise Entfernung des Sauerstoffs und des oder der Acetylene durch katalytische Umsetzung unter Einsatz eines Kupferoxid oder Ruthenium enthaltenden Katalysators erfolgt, und wobei die katalytische Umsetzung zumindest zum Teil in Form einer Hydrierung erfolgt. Eine entsprechende Anlage ist ebenfalls Gegenstand der vorliegenden Erfindung.

## Beschreibung

Die vorliegende Erfindung betrifft ein Verfahren zur Herstellung eines oder mehrerer Olefine und eine entsprechende Anlage gemäß den Oberbegriffen der unabhängigen Patentansprüche.

### Stand der Technik

Die oxidative Dehydrierung (engl. Oxidative Dehydrogenation, ODH) von Paraffinen mit zwei bis vier Kohlenstoffatomen ist grundsätzlich bekannt. Bei der ODH werden die genannten Paraffine mit Sauerstoff unter anderem zu den Olefinen gleicher Kohlenstoffzahl und zu Wasser umgesetzt.

Die ODH kann gegenüber etablierteren Verfahren zur Herstellung von Olefinen wie dem Steamcracken oder der katalytischen Dehydrierung vorteilhaft sein. So besteht aufgrund der Exothermie der beteiligten Reaktionen keine thermodynamische Gleichgewichtslimitierung. Die ODH kann bei vergleichsweise geringen Reaktionstemperaturen durchgeführt werden. Grundsätzlich ist keine Regenerierung der eingesetzten Katalysatoren erforderlich, da die Anwesenheit von Sauerstoff eine insitu-Regenerierung ermöglicht. Schließlich werden im Gegensatz zum Steamcracken geringere Mengen an wertlosen Nebenprodukten wie Koks gebildet.

Zu weiteren Details bezüglich der ODH sei auf einschlägige Fachliteratur, beispielsweise Ivars, F. und Lopez Nieto, J. M., Light Alkanes Oxidation: Targets Reached and Current Challenges, in: Duprez, D. und Cavani, F. (Hrsg.), Handbook of Advanced Methods and Processes in Oxidation Catalysis: From Laboratory to Industry, London 2014: Imperial College Press, Seiten 767-834, oder Gärtner, C.A. et al., Oxidative Dehydrogenation of Ethane: Common Principles and Mechanistic Aspects, ChemCatChem, Bd. 5, Nr. 11, 2013, Seiten 3196 bis 3217, verwiesen.

Die ODH wird auch beispielsweise in den Verfahren eingesetzt, die in der WO 2018/153831 A1, der WO 2010/115108 A1 und der DE 10 2005 000 798 A1 und der WO 2015/113747 A1 offenbart sind. Die WO 2015/113747 A1 offenbart dabei bereits eine Wasserabtrennung stromauf einer katalytischen Entfernung von Kohlenmonoxid und Sauerstoff aus einem Produktgemisch der ODH, die nochmals in der WO 2018/153831 A1 vorgeschlagen wird.

Die vorliegende Erfindung betrifft insbesondere die Herstellung von Ethylen durch ODH von Ethan (ODH-E), kann aber auch für andere Verfahrensvarianten der ODH und andere Verfahren wie z.B. die oxidative Methankopplung (engl. Oxidative Coupling of Methane, OCM), zum Einsatz kommen, bei denen sich die nachfolgend erläuterten Probleme teilweise in gleicher oder vergleichbarer Weise stellen. Bei der oxidativen Methankopplung werden ein methanreicher und ein sauerstoffreicher Strom in einen Reaktor eingespeist, wo der Sauerstoff des sauerstoffreichen Stroms und ein Teil des Methans des methanreichen Stroms unter Bildung von Wasser und Nebenprodukten zu höheren Kohlenwasserstoffen, insbesondere dem Zielprodukt Ethylen, reagieren. Die oxidative Methankopplung ist in der WO 2015/081122 A3 offenbart.

Für die nachhaltige Aktivität der in der ODH, insbesondere der ODH-E, eingesetzten Katalysatoren, bei denen es sich insbesondere MoVNbTeOₓ-Katalysatoren grundsätzlich bekannter Art handelt, ist eine minimale Konzentration von Sauerstoff erforderlich. Auf diese Weise kann eine Reduktion und damit ein Leistungsverlust der Katalysatoren vermieden werden. Daher wird in der ODH i.d.R. nicht mit einem vollständigen Sauerstoffumsatz gearbeitet und das aus einem entsprechenden Reaktor ausströmende Gasgemisch enthält Sauerstoff. Letzteres kann auch in anderen Verfahren, beispielsweise bei der OCM, der Fall sein.

Des Weiteren werden bei höheren Umsätzen in der ODH nennenswerte Mengen an Kohlenmonoxid und Kohlendioxid sowie kleine Mengen Acetylen als Nebenprodukte gebildet. Insbesondere unter industriell relevanten Reaktionsbedingungen können auch signifikante Mengen der jeweiligen Carbonsäuren der eingesetzten Paraffine als Nebenprodukte gebildet werden. Entsprechende Komponenten werden daher in einem Trennabschnitt vorteilhafterweise voneinander bzw. von dem oder den gewünschten Hauptprodukten getrennt bzw. durch chemische Umsetzung entfernt oder zu leichter entfernbaren Komponenten umgewandelt. Die vorliegende Erfindung betrifft dabei insbesondere die Entfernung von Sauerstoff und Acetylen(en) aus einem entsprechenden Gasgemisch. Auch aus einem in der OCM erhaltenen Gasgemisch sind ggf. entsprechende Komponenten enthalten und werden abgetrennt.

Aus anderen Bereichen der Technik bekannte Verfahren zur Entfernung von Acetylen aus Gasgemischen sind aus den nachfolgend noch im Detail erläuterten Gründen nicht auf die ODH bzw. die ODH-E und insoweit vergleichbare Verfahren wie die OCM übertragbar. Die Erfindung stellt sich daher die Aufgabe, Maßnahmen anzugeben, die es erlauben, aus einem insbesondere mittels ODH bzw. ODH-E oder OCM erhaltenen Gasgemisch Acetylen und Sauerstoff in vorteilhafter Weise zu entfernen. Hierzu soll eine katalytische Entfernung zum Einsatz kommen, jedoch in einer gegenüber dem zitierten Stand der Technik vorteilhaften Weise.

### Offenbarung der Erfindung

Vor diesem Hintergrund schlägt die vorliegende Erfindung ein Verfahren zur Herstellung eines oder mehrerer Olefine und eine entsprechende Anlage mit den Merkmalen der unabhängigen Patentansprüche vor. Vorteilhafte Ausgestaltungen der vorliegenden Erfindung sind jeweils Gegenstand der abhängigen Patentansprüche sowie der nachfolgenden Beschreibung.

### Vorteile der Erfindung

In einer Ausgestaltung der vorliegenden Erfindung wird eine optimierte Sequenz zur Entfernung von unerwünschten Komponenten aus einem Prozessgas eines oxidativen Verfahrens wie der ODH, insbesondere der ODH-E, aber auch beispielsweise der OCM, bereitgestellt, die einen funktionierenden, sicheren und effizienten Betrieb bei minimalen Investitionskosten ermöglicht. Bei den unerwünschten Komponenten handelt es sich um Sauerstoff und Acetylen(e). Welche Acetylene vorliegen, richtet sich insbesondere nach der Kettenlänge der eingesetzten Paraffine. In der ODH-E und der OCM handelt es sich um Acetylen (Ethin). Der Einfachheit halber ist nachfolgend auch lediglich von "Acetylen" die Rede, selbst wenn mehrere Acetylene (Alkine) vorhanden sind. Ferner ist nachfolgend stets von einer "Entfernung" von Sauerstoff und Acetylen(en) die Rede, selbst wenn diese Komponenten nur zu einem Teil, insbesondere zu einem überwiegenden Teil, d.h. insbesondere zu mehr als 90%, 95% oder 99%, entfernt werden. (Sämtliche hier verwendete Prozentangaben können sich auf den molaren Anteil, den Massenanteil oder den Volumenanteil beziehen.) Erfindungsgemäße Aspekte der vorliegenden Erfindung betreffen jedoch die Verwendung spezifischer Katalysatoren und in Ausgestaltungen von spezifischen Katalysebedingungen, die im Zusammenhang mit einem oxidativen Verfahren wie der ODH bzw. ODH-E oder der OCM für die erläuterten Zwecke besonders geeignet sind, wobei zumindest zum Teil eine Hydrierung vorgenommen wird.

Nachfolgend werden dabei zunächst die vorteilhafterweise vorgenommene Positionierung bzw. Anordnung eines Sauerstoff- und Acetylenentfernungsschritts in einer entsprechenden Trennsequenz beschrieben, anschließend nimmt die Beschreibung auf die erfindungsgemäß verwendeten Katalysatoren und die entsprechenden Katalysebedingungen Bezug. Es sei ausdrücklich betont, dass die jeweils als optional bezeichneten bzw. vorteilhafterweise bereitgestellten Merkmale nicht Teil der Erfindung sein müssen und sich die vorliegende Erfindung auch lediglich auf die jeweils als erfindungsgemäß bezeichneten Merkmale beziehen kann.

Die vorliegende Erfindung berücksichtigt durch die vorteilhafterweise vorgenommene Positionierung des Sauerstoff- und Acetylenentfernungsschritts insbesondere, dass das Einbringen von sauerstoffhaltigem Gas in eine Aminwäsche, wie sie typischerweise zur Entfernung von Kohlendioxid aus einem Prozessgas einer ODH bzw. ODH-E oder einer OCM verwendet wird, ein beträchtliches Risiko für die dauerhafte Betriebsfähigkeit derartiger Prozesseinheiten darstellt, da durch die Einbringung von Sauerstoff dort unerwünschte Nebenreaktionen auftreten können. Entsprechendes gilt allgemein für chemische Kohlendioxidwäschen, beispielsweise auch für typischerweise eingesetzte Aminwäschen.

Die vorliegende Erfindung berücksichtigt durch die vorteilhafterweise vorgenommene Positionierung des Sauerstoff- und Acetylenentfernungsschritts ferner, dass ein bestimmter Grad an Aufkonzentrierung und Partialdruckerhöhung für eine Entfernung von Acetylen vorteilhaft ist. Da Acetylene am Reaktoraustritt nur in vergleichsweise geringer Konzentration von ca. 100 bis 200 ppm Volumenanteil auftreten, ist ein bestimmter Grad an Aufkonzentrierung und Partialdruckerhöhung vorteilhaft. Umgekehrt ist die Anwesenheit nennenswerter Wassermengen nicht vorteilhaft, da diese zu weiteren Nebenreaktionen führen können.

Grundsätzlich ist zu beachten, dass im Zuge einer Trennsequenz für die Produktion von Ethylen aus einem Prozessgas aus der ODH-E (Verfahrensvarianten zur Umsetzung höherer Paraffine durch ODH und die OCM sind in gleicher Weise betroffen) ohne eine entsprechende Sauerstoffentfernung eine schrittweise Anreicherung an Sauerstoff in der Trennsequenz erfolgt, die ab einen gewissen Punkt zum Erreichen eines zündfähigen Gemischs führt. Die vorliegende Erfindung berücksichtigt durch die vorteilhafterweise vorgenommene Positionierung des Sauerstoff- und Acetylenentfernungsschritts auch dies. Die Sauerstoffentfernung erfolgt dabei vorteilhafterweise an einer Stelle der Trennsequenz, an der ein kritischer Sauerstoffgehalt noch nicht erreicht ist.

Aufgrund weiterer, im Prozessgas vorhandener Nebenprodukte ist zu erwarten, dass bei der Entfernung von Acetylen durch dessen chemische Umsetzung weitere von Ethylen bzw. von anderen Olefinen abzutrennende Komponenten entstehen. Daher wird durch die vorteilhafterweise vorgenommene Positionierung des Sauerstoff- und Acetylenentfernungsschritts sichergestellt, dass sich derartige Komponenten in der weiteren Trennsequenz ohne nennenswerten Zusatzaufwand wieder abtrennen lassen.

Für eine möglichst hohe Energieeffizienz ist es schließlich vorteilhaft, wenn die Sauerstoff- und Acetylenentfernung an einer Stelle im Prozess durchgeführt wird, bei denen die Prozessgasbedingungen nahe den für die katalytischen Reaktionen zur Sauerstoff- und Acetylenentfernung günstigsten Bedingungen liegen. Dies kann durch die vorteilhafterweise vorgenommene Positionierung des Sauerstoff- und Acetylenentfernungsschritts sichergestellt werden.

Angewandt auf die ODH-E oder die OCM erfüllt keines der für Dampfspaltverfahren bekannten Hydrierkonzepte die erläuterten Anforderungen, die jedoch durch die vorteilhafterweise vorgenommene Positionierung des Sauerstoff- und Acetylenentfernungsschritts erfüllt werden. Entsprechendes gilt jedoch auch für ein in der WO 2010/115108 A1 vorgeschlagenes Verfahren, in welchem direkt stromab des ODH-Reaktors eine Entfernung von Sauerstoff in einem separaten Reaktor erfolgt, in dem ein Hydrierkatalysator bereitgestellt wird, mittels dessen auch beispielsweise Acetylene hydriert werden können.

Durch die im Rahmen der vorliegenden Erfindung optional vorgenommene Positionierung des Sauerstoff- und Acetylenentfernungsschritts in der erläuterten Weise können die in der Reaktionseinheit gebildeten Produkte ohne konstruktiven Mehraufwand gemeinsam mit dem sonstigen Prozessgas vorhandenen Komponenten vom Hauptprodukt, im Falle der ODH-E oder der OCM Ethylen, abgetrennt werden. Hierzu werden an sich bekannte Verfahren oder Verfahrensschritte, z.B. eine Amin- oder Laugewäsche, die wässrig arbeiten und daher von dem in dem Sauerstoff- und Acetylenentfernungsschritt gebildeten Wasser nicht beeinträchtigt werden, eingesetzt.

Im Gegensatz zur WO 2010/115108 A1 stellt die Anordnung des Sauerstoff- und Acetylenentfernungsschritts stromab der wässrigen Kondensatabscheidung und des Prozessgasverdichters, wie sie im Rahmen vorteilhafterweise vorgenommen wird, beispielsweise gegenüber der eingangs zitierten WO 2018/153831 A1 einen besonderen Vorteil dar. In einer entsprechenden Anordnung können hohe Partialdrücke der umzusetzenden Komponenten und eine kompakte Bauform einer zur Sauerstoff- und Acetylenentfernung verwendeten Reaktionseinheit erreicht werden.

Zugleich werden an der vorteilhafterweise verwendeten Position noch keine ausreichenden Sauerstoffkonzentrationen für die Bildung eines zündfähigen Gemischs erreicht und die Ausgangsbedingungen nach dem Verdichter liegen in einem für die Sauerstoff- und Acetylenentfernung günstigen Prozessfenster, wie auch nachfolgend noch erläutert. Die Erfindung ermöglicht daher einen Verzicht auf anderenfalls erforderliche aufwendige Sicherheitsmaßnahmen und ist effizienter.

Somit werden durch die im Rahmen der vorliegenden Erfindung optional vorgeschlagene Anordnung sämtliche zuvor erläuterten Randbedingungen erfüllt und ein signifikanter Vorteil im Anlagendesign erzielt. Die optional vorgesehene Positionierung des Sauerstoff- und Acetylenentfernungsschritts ist jedoch nicht erfindungswesentlich, insbesondere dann, wenn entsprechende Bedingungen auch an anderer Stelle einer entsprechenden Trennsequenz geschaffen werden können.

Wie bereits erwähnt, werden erfindungsgemäß spezifische Katalysatoren und insbesondere zugehörige vorteilhafte Katalysebedingungen zur Entfernung von Acetylen(en) und Sauerstoff aus einem entsprechenden Gasgemisch vorgeschlagen. Wie ebenfalls bereits erwähnt, sind aus anderen Bereichen der Technik bekannte Verfahren zur Entfernung von Acetylen aus Gasgemischen bei der ODH bzw. der ODH-E nicht oder nicht ohne weiteres einsetzbar.

Beispielsweise kann zur Entfernung von Acetylen aus einem Prozessgas eines Dampfspaltverfahrens eine isotherme Rohgashydrierung durchgeführt werden. Zu Dampfspaltverfahren allgemein sei in diesem Zusammenhang beispielsweise auf Fachliteratur wie den Artikel "Ethylene" in Ullmann's Encyclopedia of Industrial Chemistry, Onlineausgabe, 2009, DOI 10.2002/14356007.a10_045.pub3, und zur isothermen Rohgashydrierung insbesondere auf Falqi, F.: "The Miracle of Petrochemicals. Olefins Industry: An In-Depth Look at Steam-Crackers", Universal-Publishers 2009, ISBN 1-59942-915-2, Abschnitt "The Linde Raw Gas Hydrogenation System", Seiten 20 bis 22, verwiesen.

Die isotherme Rohgashydrierung erfolgt insbesondere nach der Trocknung des Rohgases und vor der Abtrennung von Kohlenwasserstoffen mit zwei bzw. drei Kohlenstoffatomen. Auch eine Hydrierung von in einer entsprechenden Trennung gebildeten Fraktionen ist jedoch grundsätzlich möglich, beispielsweise eine isotherme Hydrierung einer Fraktion von Kohlenwasserstoffatomen mit zwei und ggf. mehr Kohlenstoffatomen nach einer Deethanisierung und vor einer Demethanisierung, oder eine adiabate Hydrierung einer Fraktion von Kohlenwasserstoffatomen mit zwei Kohlenstoffatomen vor der Bildung einer Ethan- und einer Ethylenfraktion.

In Dampfspaltverfahren bzw. in diesen nachgeschalteten Schritten erfolgen die erwähnten Hydrierungen stets in Abwesenheit von molekularem Sauerstoff und bei Kohlenmonoxidgehalten von weniger als 1%, typischerweise weniger als 1000 ppm Volumenanteil. Für die selektive Acetylenentfernung in ethylenreichen Strömen werden dabei typischerweise Edelmetallkatalysatoren, beispielsweise auf Palladiumbasis, verwendet. Diese können ggf. mit weiteren Edelmetallen dotiert sein. Auch die Verwendung von Nickelkatalysatoren, welche jedoch sehr sensitiv auf den Kohlenmonoxidgehalt in der Matrix reagieren und daher nur bei Kohlenmonoxidgehalten von ca. 50 bis maximal 5.000 ppm Volumenanteil eingesetzt werden, ist bekannt.

Insgesamt lässt sich feststellen, dass die herkömmlicherweise in Dampfspaltverfahren eingesetzten Katalysatoren für die Selektivhydrierung wie die erwähnten Edelmetall- oder Nickelkatalysatoren aufgrund des vergleichsweise hohen Sauerstoffgehalts in einem Gasgemisch aus der ODH bzw. ODH-E oder der OCM nicht verwendet werden können. Grund hierfür ist insbesondere, dass durch die zusätzliche Hydrierung von Sauerstoff in den herkömmlichen Verfahren die Temperatur im Katalysatorbett durch die adiabate Reaktionsführung so stark ansteigen würde, dass die Hydrierung von Ethylen stark beschleunigt wird und somit ein hoher Anteil des Produktes verloren geht. Im ungünstigsten Fall kommt es zum Totalumsatz des Wasserstoffs und zu einem unkontrollierten Durchgehen des Reaktors.

Ferner ist zu erwarten, dass konventionelle Katalysatoren von dem bei der Umsetzung von Sauerstoff gebildeten Wasser entweder vergiftet werden und somit nur geringe Standzeiten aufweisen, oder aber die Bildung unerwünschter Nebenprodukte (Polymerisat, sogenanntes Grünöl) stark begünstigt wird.

Konventionelle Edelmetallkatalysatoren weisen ferner typischerweise eine geringe Toleranz gegenüber Kohlenmonoxid auf, wie es in einem Gasgemisch aus der ODH bzw. ODH-E oder der OCM ebenfalls enthalten ist. Insbesondere bei höheren Kohlenmonoxidkonzentrationen von mehreren tausend ppm Volumenanteil geht die Aktivität sehr stark zurück. Eine Kompensation durch Temperaturerhöhung und ohne signifikante Selektivitätseinbußen ist hier nur bedingt möglich.

Für die Sauerstoff- und Acetylenentfernung zur Aufarbeitung von Gasgemischen aus fluidkatalytischen Spaltverfahren (Fluid Catalytic Cracking, FCC) kommen typischerweise sulfidierte Kupfer- oder Nickelkatalysatoren zum Einsatz, wodurch eine Möglichkeit zum Schwefeleintrag in das Reaktionsgas besteht bzw. sogar eine fortlaufende Schwefelung erforderlich ist. Generell ist die Verwendung von kupferbasierten Katalysatoren für die Sauerstoff- und/oder Acetylenentfernung aus Gasgemischen aus petrochemischen Prozessen auch beispielsweise aus der US 5,446,232 A, der US 4,034,062 A, der US 2,953,608 A, der US 3,912,789 A, der US 4,049,743 A, der US 4,035,433 A und der US 2,381,707 A bekannt. Die im Rahmen der vorliegenden Erfindung vorgeschlagenen Maßnahmen sind jedoch nicht bekannt oder durch diesen Stand der Technik nahegelegt.

Eine kombinierte Entfernung von Acetylen und Sauerstoff direkt am Austritt eines ODH-E-Reaktors ist, wie zuvor erwähnt, beispielsweise aus der WO 2010/115108 A1 bekannt. Auch hier sind die vorgeschlagenen Maßnahmen aber nicht beschrieben. Sulfidierte Nickel- oder Kupferkatalysatoren für die Aufreinigung von Gasgemischen aus der FCC benötigen typischerweise die erwähnte kontinuierliche Schwefelzugabe (beispielsweise in Form von Dimethyldisulfid, DMDS) um die Aktivität und Selektivität konstant zu halten. Dies würde für die ODH bzw. ODH-E jedoch bedeuten, dass man eine neue Verunreinigung zugeben muss, die wiederum das Produkt verunreinigen könnte. Der Einsatz von Nickelkatalysatoren bei vergleichsweise hohem Kohlenmonoxidgehalt ist aufgrund der möglichen Bildung von flüchtigen Nickelcarbonylen ebenfalls als kritisch anzusehen.

Die erfindungsgemäß eingesetzten Katalysatoren weisen sämtliche zuvor erläuterten Nachteile herkömmlicher Katalysatoren nicht auf, insbesondere wenn sie unter den angegebenen Katalysebedingungen eingesetzt werden. Die erfindungsgemäß eingesetzten Katalysatoren und ggf. Katalysebedingungen entfalten ihre vorteilhaften Wirkungen dabei insbesondere in Kombination mit der vorteilhafterweise vorgenommenen Positionierung des Sauerstoff- und Acetylenentfernungsschritts. Es ist jedoch auch möglich, die erfindungsgemäß vorgeschlagenen Katalysatoren und ggf. Katalysebedingungen an anderer Position in der Trennsequenz einzusetzen.

Insgesamt schlägt die vorliegende Erfindung in Anbetracht der erläuterten Umstände ein Verfahren zur Herstellung eines oder mehrerer Olefine vor, bei dem ein Reaktionseinsatz gebildet wird, der Sauerstoff und ein oder mehrere Paraffine enthält. Im Falle der ODH-E enthält der Reaktionseinsatz dabei im Wesentlichen Ethan als Paraffin, weitere Paraffine sind nicht oder nur in geringem Anteil enthalten. Auch das in der OCM verwendete Methan stellt ein Paraffin in diesem Sinne dar. Wird eine ODH höherer Paraffine durchgeführt, weisen diese insbesondere drei oder vier Kohlenstoffatome auf.

Im Rahmen der vorliegenden Erfindung wird ferner ein Teil des Sauerstoffs in dem Reaktionseinsatz mit einem Teil des unter der Paraffine unter Erhalt eines Prozessgases durch ein oxidatives Verfahren, insbesondere durch oxidative Dehydrierung oder oxidative Kopplung, zu dem oder zu den Olefinen umgesetzt. Wiederum erfolgt bei der ODH-E und OCM eine Umsetzung zu Ethylen, es werden also allenfalls geringere Mengen anderer Olefine gebildet. In entsprechender Weise werden bei der ODH höherer Paraffine vorzugsweise die jeweils kettenlängengleichen Olefine gebildet. Das Prozessgas enthält zumindest den nicht umgesetzten Teil des oder der Paraffine und des Sauerstoffs, das oder die Olefine, ein oder mehrere Acetylene, Kohlenmonoxid, Kohlendioxid und Wasser. Diese Aufzählung ist nicht abschließend. Insbesondere kann ein entsprechendes Prozessgas die zuvor erläuterten Nebenprodukte zusätzlich enthalten, insbesondere Carbonsäuren mit der gleichen Kettenlänge wie die eingesetzten Paraffine.

Das erfindungsgemäße Verfahren umfasst, dass das Prozessgas oder ein Gasgemisch, das unter Verwendung zumindest eines Teils des Prozessgases gebildet wird, teilweise oder vollständig einer zumindest teilweisen Entfernung des Sauerstoffs und des oder der Acetylene unterworfen wird, wobei die zumindest teilweise Entfernung des Sauerstoffs und des oder der Acetylene durch katalytische Umsetzung unter Einsatz eines Kupferoxid oder Ruthenium enthaltenden Katalysators erfolgt und wobei die katalytische Umsetzung zumindest zum Teil in Form einer Hydrierung erfolgt. Wird ein Kupferoxid enthaltender Katalysator eingesetzt, enthält dieser vorteilhafterweise auch Manganoxid. Durch den Einsatz dieser Katalysatoren können die zuvor ausführlich erläuterten Vorteile erzielt werden Die zumindest teilweise Entfernung des Sauerstoffs und des oder der Acetylene durch die katalytische Umsetzung erfolgt dabei insbesondere in einem verfahrenstechnischen Schritt, d.h. in nur einer Reaktionseinheit und/oder unter Verwendung nur eines Katalysators bzw. Katalysatorbetts. Es wird also insbesondere gleichzeitig der Gehalt an Sauerstoff und an dem oder den Acetylenen reduziert.

Die zumindest teilweise Entfernung des Sauerstoffs und des oder der Acetylene durch katalytische Umsetzung kann in Ausgestaltungen der vorliegenden Erfindung insbesondere durch Hydrieren des Sauerstoffs und des oder der Acetylene erfolgen, wobei in eine entsprechende Reaktionseinheit optional Wasserstoff eingespeist werden kann. Gemäß alternativen Ausgestaltungen ist jedoch eine zumindest teilweise oxidative Entfernung zumindest eines Teils des Sauerstoffs vorgesehen, bei der das in einem entsprechenden Gasgemisch enthaltene Kohlenmonoxid mit dem Sauerstoff unter Bildung von Kohlendioxid oxidiert wird. Je nach freigesetzter Reaktionswärme können für die Sauerstoff- und Acetylenentfernung eine isotherme Reaktionseinheit oder eine mindestens einstufige adiabate Reaktionseinheit eingesetzt werden. Die ggf. im Fall einer Hydrierung, aber auch bei einer oxidativen Entfernung ggf. eingespeiste Wasserstoffmenge und/oder das Temperaturniveau werden derart eingestellt, dass eine möglichst vollständige Umsetzung von Sauerstoff und Acetylen(en) erreicht werden. Zu erwartende Produkte dieser Umsetzung sind dabei insbesondere weiteres Ethylen, Ethan, Kohlenmonoxid, Kohlendioxid und Wasser sowie ggf. Spuren von Methan, Oxygenaten und sogenanntes Grünöl. Diese Komponenten lassen sich in vorhandenen Trennschritten einfach entfernen.

Die zumindest teilweise Entfernung des Sauerstoffs und des oder der Acetylene erfolgt vorteilhafterweise, nachdem das Prozessgas oder ein Teil hiervon teilweise oder vollständig einer Kondensatabscheidung und einer Verdichtung unterworfen wurde und bevor ein nach der zumindest teilweisen Entfernung von Sauerstoff und Acetylen(en) verbleibendes Gasgemisch zumindest teilweise einer oder mehreren Stufen einer Kohlendioxidentfernung unterworfen wird. Auf diese Weise können die zuvor erläuterten Vorteile erzielt werden können.

In einer Variante der Erfindung, in der die erfindungsgemäße Positionierung der Sauerstoff- und Acetylenentfernung nach der Kondensatabscheidung und der Verdichtung vorgenommen wird, kann eine mehrstufige Kohlendioxidentfernung vorgesehen sein, wobei einzelne Stufen der Kohlendioxidentfernung auch stromauf der Sauerstoff- und Acetylenentfernung durchgeführt werden können. Mit anderen Worten kann die zumindest teilweise Entfernung des Sauerstoffs und des oder der Acetylene stromab einer oder mehrerer Stufen der Kohlendioxidentfernung und stromauf einer oder mehrerer weiterer Stufen der Kohlendioxidentfernung durchgeführt werden.

Vorteilhafterweise werden stromab der zumindest teilweise Entfernung des Sauerstoffs und des oder der Acetylene eine Trocknung und ein oder mehrere Trennschritte durchgeführt. In dem oder den Trennschritten können die bei der zumindest teilweisen Entfernung des Sauerstoffs und des oder der Acetylene gebildeten Komponenten in dem verbleibenden Prozessgas bzw. einem entsprechenden Folgegemisch einfach abgetrennt werden, ohne dass hier zusätzliche Anlagenteile erforderlich sind.

Der oder die nachgeschalteten Trennschritte sind insbesondere derart ausgestaltet, dass sie nicht nicht nur die bei der erfindungsgemäß vorgesehenen Entfernung von Sauerstoff und Acetylen gebildeten (Neben-)Produkte entfernen, sondern auch sonstige unerwünschte Komponenten wie Restkohlendioxid, Restsauerstoff sowie ggf. vorhandenes Methan und/oder andere Leichtsieder.

Die Entfernung von Sauerstoff- und Acetylen schafft im Rahmen der vorliegenden Erfindung insbesondere die Grundvoraussetzungen dafür, dass diese weiteren Trennschritte (insbesondere bei kryogener Destillation) sicher durchgeführt werden können. Die Verwendung solcher nachgeschalteten Schritte ermöglicht es auch, dass stromauf keine vollständige Entfernung von Sauerstoff erfolgen werden muss. Wie zuvor erwähnt, wird unter einer "Entfernung" hier auch eine teilweise Entfernung verstanden. Durch den Einsatz der vorliegenden Erfindung wird insbesondere der Vorteil erzielt, dass es weiter stromab in aufkonzentrierten Leichtsiederströmen nicht zur Bildung von zündfähigen Gemischen kommt.

Insbesondere die vollständige Entfernung von Kohlendioxid braucht im Rahmen der vorliegenden Erfindung nicht bereits stromauf erfolgen sondern diese kann über entsprechende nachgeschalteten Trennschritte vorgenommen werden.

Wahlweise können im Rahmen der vorliegenden Erfindung der oder zumindest einer der erwähnten Trennschritte kryogen und/oder adsorptiv durchgeführt werden. Insbesondere kann eine kryogene Destillation erfolgen; es ist jedoch auch die Verwendung von beispielsweise alternativen Reinigungsschritten wie Druckwechseladsorption möglich.

Wie erwähnt, liegt das Prozessgas oder dessen entsprechend bearbeiteter Teil bzw. ein unter Verwendung des Prozessgases gebildetes Gasgemisch in den erläuterten Ausgestaltungen, in denen die angegebene Positionierung der Sauerstoff- und Acetylenentfernung vorgenommen wird, stromauf der Sauerstoff- und/oder Acetylenentfernung unter besonders günstigen Bedingungen vor. Diese Bedingungen werden nachfolgend unter Bezugnahme auf die vorteilhaften Katalysebedingungen für die unterschiedlichen Katalysatoren erläutert. Entsprechende Bedingungen können aber auch ohne die erwähnte Positionierung der Sauerstoff- und Acetylenentfernung nach der Kondensatabscheidung und der Verdichtung vorliegen oder entsprechend geschaffen werden.

Insbesondere durch den Einsatz der erwähnten Katalysatoren und der vorteilhafterweise eingesetzten Katalysebedingungen kann eine vollständige oder nahezu vollständige Umsetzung von Sauerstoff und Acetylen erzielt werden, wobei sich zugleich nur minimale Verluste an Ethylen ergeben und eine minimale Bildung von Nebenprodukten wie beispielsweise Grünöl und/oder Carbonsäuren erfolgt. In Ausgestaltungen der Erfindung wird eine besonders hohe Stabilität und Standzeit des Katalysators erzielt. Im Gegensatz zur WO 2018/153831 A1 erfolgt dabei die katalytische Umsetzung zumindest zum Teil in Form einer Hydrierung und insbesondere unter Zudosierung von Wasserstoff.

In einer Ausgestaltung der vorliegenden Erfindung wird der Kupferoxid enthaltende Katalysator eingesetzt, der insbesondere auch Manganoxid enthalten kann. Ein vorteilhaft einsetzbarer Katalysator weist dabei im Rahmen der vorliegenden Erfindung insbesondere 7 bis 11 % Kupfer- und 10 bis 15 % Manganoxid . Ein entsprechender Katalysator kann insbesondere auf Körpern aus geeigneten Trägermaterialen geträgert sein, beispielsweise aus Aluminiumoxid. Weitere Eigenschaften des Kupferoxid enthaltenden Katalysators umfassen, dass die Katalysatorkörper verschiedene Formen und Strukturen wie Tabletten, Ringe, Dreifachringe (Triholes) sowie andere übliche Formen und Strukturen aufweisen, wobei sich die ausgewählte Form an die im Verfahren entsprechenden Anforderungen, z.B. der Minimierung des Druckverlusts über den katalytischen Reaktor, angepasst ist.

In dieser Ausgestaltung, in der der Kupferoxid enthaltende Katalysator eingesetzt wird, umfasst die zumindest teilweise Entfernung des Sauerstoffs und des oder der Acetylene eine katalytische Hydrierung zumindest eines Teils des Sauerstoffs. Die zumindest teilweise Entfernung des Sauerstoffs und des oder der Acetylene wird im Rahmen der Erfindung insbesondere bei Reaktionsbedingungen durchgeführt, die eine Temperatur von 180 bis 360 °C, insbesondere von 200 bis 250 °C, weiter insbesondere 220 bis 240 °C, einen Druck von 1 bis 30 bar abs., insbesondere von 10 oder 15 bis 25 bar (abs.), eine stündliche Gas-Raumgeschwindigkeit (Gas Hourly Space Velocity, GHSV) von 1.000 bis 15.000 h⁻¹, insbesondere von 2.000 bis 5.000 h⁻¹, weiter insbesondere von 3.000 bis 4.000 h⁻¹, und ein Verhältnis von Wasserstoff zu Sauerstoff von 0 bis 5 umfassen. Insbesondere kann bei der zumindest teilweisen Umsetzung des Sauerstoffs das Verhältnis von Wasserstoff zu Sauerstoff zu dessen Hydrierung in einem Bereich von beispielsweise 1 bis 4 oder 2 bis 3 liegen. Es handelt sich hierbei insbesondere um Molverhältnisse unter den genannten Bedingungen. Die verwendeten Drücke richten sich auch nach der mehrfach erläuterten Positionierung des Sauerstoff- und Acetylenentfernungsschritts.

Im Rahmen der vorliegenden Erfindung wurde überraschenderweise erkannt, dass sich unter den zumindest teilweise hydrierenden Bedingungen bezüglich Sauerstoff auch eine Umsetzung von Acetylen ergibt. Durch die Verwendung dieser Bedingungen und des eingesetzten Katalysators kann daher eine gleichzeitige Umsetzung von Sauerstoff und Acetylen erfolgen. Ohne in irgendeiner Weise an diese Erläuterungen gebunden zu sein, könnte eine Erklärung für diese Umsetzung darin liegen, dass sich bei den verwendeten Temperaturen Acetylen am Katalysator zersetzt und mit Sauerstoff zu Kohlenmonoxid bzw. Kohlendioxid weiterreagiert. Wenngleich also ein bezüglich Sauerstoff hydrierender Katalysator verwendet wird, ergibt sich dennoch bezüglich Acetylen eine entsprechende Umsetzung.

Alternativ zu der zumindest teilweisen hydrierenden Umsetzung könnte grundsätzlich auch eine oxidative Umsetzung des Sauerstoffs über die Umsetzung mit dem im Produktgas enthaltenen Kohlenmonoxid durchgeführt werden, wie in der WO 2018/153831 A1 erwähnt. Als Produkt wird dabei Kohlendioxid gebildet. Typischerweise zersetzt sich das anwesende Acetylen jedoch hierbei auf der Katalysatoroberfläche und führt zur Verkokung mit einhergehendem raschen Aktivitätsverlust des Katalysators über die Zeit. Die Erfindung vermeidet durch die genannten Reaktionsbedingungen diesen Nachteil.

Im Rahmen der vorliegenden Erfindung konnte somit überraschenderweise gezeigt werden, dass bei geeigneten Reaktionsbedingungen bei der Verwendung des Kupferoxid enthaltenden Katalysators, insbesondere eines auf Kupfer- und Manganoxid basierenden Katalysators, auch simultan Acetylen ohne nennenswerte Aktivitätsverluste über der Zeit entfernt werden kann. Eine Zudosierung von Wasserstoff kann eine Verkokung durch die Anwesenheit von Acetylen nochmals verringern. Mit anderen Worten werden im Rahmen der vorliegenden Erfindung anstelle von oxidierenden Bedingungen solche Bedingungen eingesetzt, die zu einer zumindest teilweisen Hydrierung des Sauerstoffs führen.

In einer alternativen Ausgestaltung der vorliegende Erfindung wird der Ruthenium enthaltende Katalysator eingesetzt. Ein vorteilhaft einsetzbarer Katalysator weist dabei im Rahmen der vorliegenden Erfindung insbesondere 0,01 bis 1 % Ruthenium auf. Ein entsprechender Katalysator kann insbesondere auf Körpern aus geeigneten Trägermaterialen geträgert sein, beispielsweise aus Aluminiumoxid. Weitere Eigenschaften umfassen, dass die Katalysatorkörper verschiedene Formen und Strukturen wie Tabletten, Ringe, Dreifachringe (Triholes) sowie andere übliche Formen und Strukturen aufweisen, wobei sich die ausgewählte Form an die im Verfahren entsprechenden Anforderungen, z.B. der Minimierung des Druckverlusts über den katalytischen Reaktor, angepasst ist.

Beim Einsatz des Ruthenium enthaltenden Katalysators umfasst die zumindest teilweise Entfernung des Sauerstoffs und des oder der Acetylene vorteilhafterweise eine katalytische Hydrierung, die bei Reaktionsbedingungen durchgeführt wird, die eine Temperatur von 120 bis 300 °C, insbesondere von 130 bis 170 °C, einen Druck von 1 bis 30 bar abs., insbesondere von 10 bis 25 bar abs., eine stündliche Gas-Raumgeschwindigkeit von 1.500 bis 4.500 h⁻¹ und ein Verhältnis von Wasserstoff zu Sauerstoff von 1 bis 14, beispielsweise von 4 bis 10, umfassen. Die verwendeten Drücke richten sich auch hier nach der mehrfach erläuterten Positionierung des Sauerstoff- und Acetylenentfernungsschritts.

Es hat sich im Rahmen der vorliegenden Erfindung herausgestellt, dass auch bekannte Ruthenium enthaltende Katalysatoren für die simultane Hydrierung von Sauerstoff und Acetylen in dem vorliegenden Einsatzgebiet vorteilhaft sind. Sie weisen eine hohe Toleranz gegen den eingangs erwähnten starken adiabaten Temperaturanstieg auf. Auch der Ethylenverlust von unter 2% ist tolerabel.

In allen Fällen kann im Rahmen der vorliegenden Erfindung die zumindest teilweise Entfernung des Sauerstoffs und des oder der Acetylene unter Zugabe von Wasserstoff durchgeführt werden, und zwar entweder, um für die Hydrierungsreaktionen geeignete Reaktionsbedingungen einzustellen, oder um bei der Oxidation von Kohlenmonoxid mit Sauerstoff, wie erwähnt, auch die geringfügige Zersetzung von Acetylen zu vermeiden.

Wie bereits erwähnt, kann die vorliegende Erfindung insbesondere im Verfahren zur oxidativen Dehydrierung von Ethan eingesetzt werden, wobei sich die zuvor erläuterten Zusammensetzungen des Einsatzgemischs und des Prozessgases einstellen bzw. vorgegeben werden.

Die vorliegende Erfindung erstreckt sich auch auf eine Anlage zur Herstellung eines oder mehrerer Olefine, bezüglich derer auf den entsprechenden unabhängigen Patentanspruch verwiesen wird. Zu Merkmalen und Vorteilen dieser Anlage, die vorteilhafterweise zur Durchführung eines Verfahrens eingerichtet ist, wie es zuvor in Ausgestaltungen erläutert wurde, sei auf die Ausführungen oben verwiesen.

Die Erfindung wird nachfolgend unter Bezugnahme auf die beigefügte Zeichnung sowie auf erfindungsgemäße Ausführungsbeispiele und Gegenbeispiele näher erläutert.

### Ausführungsbeispiele

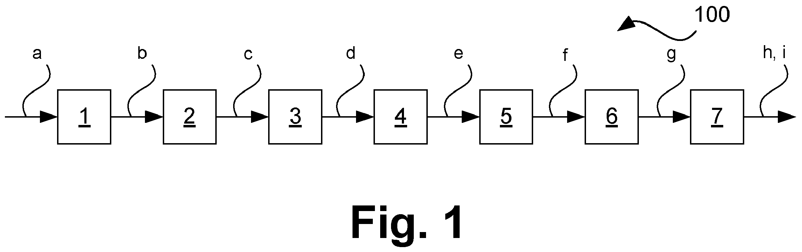

In Figur 1 ist ein Verfahren gemäß einer besonders bevorzugten Ausführungsform der vorliegenden Erfindung veranschaulicht und insgesamt mit 100 bezeichnet. Die Erläuterungen bezüglich des Verfahrens 100 gelten gleicherweise auch für eine entsprechende Anlage, in welcher die in Figur 1 dargestellten Verfahrensschritte durch entsprechende Anlagenkomponenten realisiert werden.

In dem Verfahren 100 wird ein Reaktionseinsatz gebildet, der Sauerstoff und ein oder mehrere Paraffine enthält, und in Form eines Stoffstroms a einer oxidativen Dehydrierung 1 unterworfen. Ein in der oxydativen Dehydrierung gebildetes Prozessgas wird zumindest teilweise einer Kondensatabtrennung 2 zugeführt, in welcher sich beispielsweise Wasser und Essigsäure kondensativ abscheiden. Das entsprechende Prozessgas oder dessen Teil wird der Kondensatabtrennung in Form eines Prozessgasstroms b zugeführt.

Das aus der Kondensatabtrennung entnommene, an Wasser und ggf. weiteren Komponenten abgereicherte Prozessgas wird in Form eines Prozessgasstroms c einem Prozessgasverdichter bzw. Rohgasverdichter 3 zugeführt und dort auf ein Druckniveau von beispielsweise mehr als 15 bar verdichtet. Der verdichtete Prozessgasstrom wird in Form eines Stoffstroms d einer zumindest teilweisen Entfernung 4 von Sauerstoff und Acetylenen zugeführt, in der durch die Einstellungen bestimmter Reaktionsbedingungen sowohl Acetylene als auch Sauerstoff umgesetzt werden. Das entsprechend bearbeitete Prozessgas wird in Form eines Prozessgasstroms e einer Kohlendioxidabtrennung 5 unterworfen, gelangt anschließend in Form eines Prozessgasstroms f in eine Trocknung 6 und wird schließlich in Form eines Prozessgasstroms g einem oder mehreren weiteren Trennschritten 7 unterworfen, die hier stark vereinfacht dargestellt sind. In dem oder den Trennschritten 7 werden ein oder mehrere Fraktionen h, i gebildet und aus dem Verfahren 100 ausgeführt.

Grundsätzlich kann das Verfahren 100 das in Figur 1 veranschaulicht ist in unterschiedlicher Ausgestaltung realisiert werden. Insbesondere können die Verfahrensschritte 5 bis 7 in abweichender Anordnung durchgeführt werden, es kann eine Rückführung von Teilströmen oder Fraktionen erfolgen und dergleichen. Insbesondere kann auch die Sauerstoff- und Acetylenentfernung 4 an anderer Stelle als in Figur 1 veranschaulicht vorgesehen sein.

Gemäß einem Beispiel 1 wurde ein kommerziell verfügbarer Katalysator bestehend aus auf Aluminiumoxid geträgertem Kupfer- und Manganoxid auf seine Eignung zum Einsatz bei der Sauerstoff- und Acetylenentfernung aus einem Prozessgas der ODH bzw. ODH-E untersucht. Der Katalysator wurde auf 3 mm zerkleinert und in einem Rohrreaktor mit einem Innendurchmesser von 29 mm eingefüllt. Oberhalb der Katalysatorschüttung wurden Glasperlen als Inertmaterial eingefüllt. Ein Katalysatorbett von 15 cm wurde realisiert. Der Reaktor wurde als adiabater Rohrreaktor betrieben und über Heizbänder beheizt, um Wärmeverluste auszugleichen. Über Massendurchflussregler wurden Gasgemische mit der in Tabelle 1A angegebenen Zusammensetzung (in Volumenprozent) zugespeist:

**Tabelle 1A**

| | **Gasgemisch 1** | **Gasgemisch 2** |
|---|---|---|
| Wasserstoff | 0 | 0,66 |
| Ethylen | 35,9 | 35,9 |
| Acetylen | 0,015 | 0,015 |
| Ethan | 59,1 | 52,5 |
| Sauerstoff | 0,47 | 0,47 |
| Stickstoff | 1,77 | 7,7 |
| Kohlenmonoxid | 2,72 | 2,72 |

In den Tabellen 1B und 1C ist die erfolgreiche simultane Entfernung von Sauerstoff und Acetylen über eine Laufzeit von mehr als 250 Stunden für die beiden in Tabelle 1A angegebenen Gasgemische belegt. Zwischen 158,8 Stunden und 179,2 Stunden wurde dabei zwischen Gasgemisch 1 und 2 gemäß Tabelle 1A umgeschaltet, also zusätzlich auch Wasserstoff zugegeben. Die beiden Tabellen 1B und 1C betreffen also einen kontinuierlich durchgeführten Versuch.

Als Reaktionsbedingungen wurden eine stündliche Gas-Raumgeschwindigkeit (Gas Hourly Space Velocity, GHSV) von ca. 3.700 h⁻¹, eine Reaktoreintrittstemperatur von 230 °C und ein Druck von 20 bar verwendet. Es zeigt sich, dass Sauerstoff sowohl über Oxidation von Kohlenmonoxid (in Abwesenheit von Wasserstoff, Gasgemisch 1) als auch über eine Hydrierung (Gasgemisch 2) entfernt werden kann. Die Ethylenverluste sind jeweils ausgesprochen gering.

**Tabelle 1B (Gasgemisch 1 gemäß Tabelle 1A)**

| | | | | | |
|---|---|---|---|---|---|
| Laufzeit | 4,7 | 58,6 | 118,5 | 140,1 | 158,8 |
| Ethylenverlust | 1,9 | 0,15 | 0,08 | 0,28 | 0,05 |
| Sauerstoffumsatz | 100 | 100 | 100 | 100 | 100 |
| Acetylen umsatz | 100 | 100 | 100 | 100 | 100 |

**Tabelle 1C (Gasgemisch 2 gemäß Tabelle 1A)**

| | | | | |
|---|---|---|---|---|
| Laufzeit | 179,2 | 199,2 | 226,2 | 254,1 |
| Ethylenverlust | 0,00 | 0,00 | 0,00 | 0,00 |
| Sauerstoffumsatz | 99,8 | 100 | 100 | 100 |
| Acetylen umsatz | 100 | 100 | 100 | 100 |

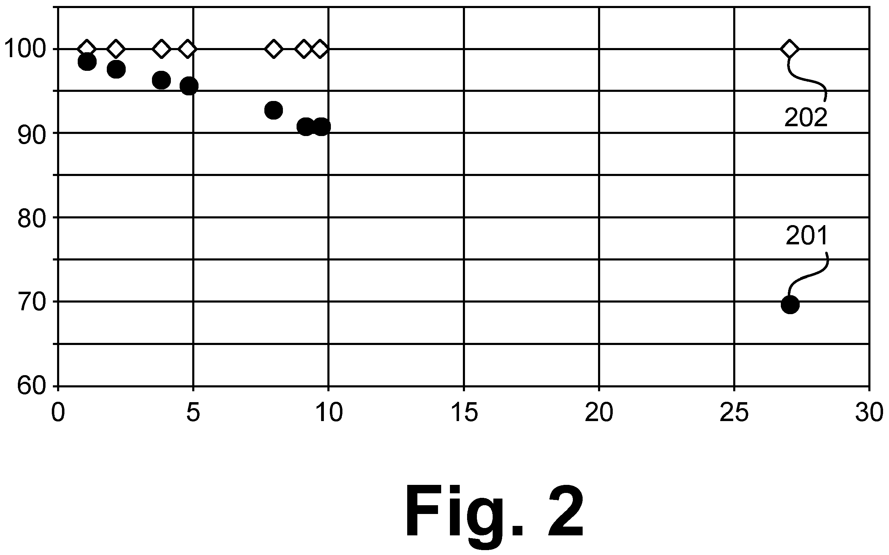

In einem Gegenbeispiel 1 wurden derselbe Versuchsaufbau wie gemäß Beispiel 1 verwendet und dieselbe GHSV eingesetzt. Es wurde jedoch lediglich mit einer Reaktoreintrittstemperatur von 170 °C gearbeitet. Wie aus Figur 2 ersichtlich, wird der Katalysator unter diesen Bedingungen sehr rasch deaktiviert und der Umsatz von Sauerstoff nimmt ab. Die Figur 2 zeigt dabei eine Versuchszeit in Stunden auf der Abszisse gegenüber einer Umsetzung in Molprozent auf der Ordinate. Die Umsetzung von Sauerstoff ist mit 201 und die Umsetzung von Acetylen mit 202 veranschaulicht.

Gemäß Beispiel 2 wurde ein Muster eines kommerziell verfügbaren Katalysators mit geträgertem Ruthenium auf Aluminiumoxid untersucht. Die Kugeln (2 bis 4 mm Durchmesser) wurden in einem Rohrreaktor mit einem inneren Durchmesser von 29 mm eingefüllt. Oberhalb der Katalysatorschüttung wurden Glasperlen als Inertmaterial eingefüllt. Ein Katalysatorbett von 20 cm wurde realisiert. Der Reaktor wurde über Heizbänder beheizt. Der Reaktor wird als adiabater Rohrreaktor betrieben. Über Massendurchflussregler wurde ein Gasgemisch mit der in Tabelle 2A angegebenen Zusammensetzung (in Volumenprozent) zugespeist:

**Tabelle 2A**

| | **Gasgemisch** |
|---|---|
| Wasserstoff | 2,06 |
| Ethylen | 34,70 |
| Acetylen | 0,017 |
| Ethan | 39,20 |
| Sauerstoff | 0,49 |
| Stickstoff | 30,65 |
| Kohlenmonoxid | 2,89 |

In Tabelle 2B ist die erfolgreiche simultane Entfernung von Sauerstoff und Acetylen bei unterschiedlichen Bedingungen gezeigt. Es wurde jeweils ein Druck von 20 bar im Reaktor eingestellt.

**Tabelle 2B**

| | | | | |
|---|---|---|---|---|
| GHSV | 2084 | 4340 | 4297 | 2510 |
| Eintrittstemperatur | 152 | 150 | 189 | 152 |
| Ethylenverlust | 1,9 | 0,7 | 0,1 | 0,8 |
| Sauerstoffumsatz | 99,2 | 97,7 | 97,3 | 96,9 |
| Acetylen umsatz | 100 | 100 | 100 | 100 |

In einem Gegenbeispiel 2 wurde derselbe Katalysator wie in Beispiel 2 in derselben Versuchsapparatur mit einem Katalysatorbett von 30 cm getestet. Dabei wurden die in Tabelle 2C angegebenen Gasgemische (Angaben in Volumenprozent) eingestellt.

**Tabelle 2C**

| | **Gasgemisch 1** | **Gasgemisch 2** | **Gasgemisch 3** |
|---|---|---|---|
| Wasserstoff | 8,36 | 7,82 | 12,41 |
| Ethylen | 37,30 | 35,13 | 35,31 |
| Acetylen | 0,016 | 0,007 | 0,015 |
| Ethan | 48,90 | 53,37 | 45,43 |
| Sauerstoff | 0,44 | 0,502 | 0,732 |
| Stickstoff | 2,05 | 1,95 | 3,32 |
| Kohlenmonoxid | 2,92 | 1,22 | 2,77 |

In Gegenbeispiel 2 wurde ein Druck von 24 bar verwendet. Die Ergebnisse für die drei in Tabelle 2C angegebenen Gasgemische sind in Tabelle 2D veranschaulicht. Wie aus Tabelle 2D ersichtlich, sind unter den angegebenen Bedingungen, insbesondere bei dem hohen Wasserstoff/Sauerstoff-Verhältnis, die Ethylenverluste sehr hoch.

**Tabelle 2D**

| | Gemisch 1 | Gemisch 2 | Gemisch 3 |
|---|---|---|---|
| GHSV | 1927 | 2449 | 1961 |
| Eintrittstemperatur | 185,5 | 155 | 158,5 |
| Ethylenverlust | 3,2 | 4,2 | 5,5 |
| Sauerstoffumsatz | 100 | 100 | 100 |
| Acetylen umsatz | 98,7 | 99,1 | 99,6 |

## Patentansprüche

1. Verfahren (100) zur Herstellung eines oder mehrerer Olefine, bei dem ein Reaktionseinsatz gebildet wird, der Sauerstoff und ein oder mehrere Paraffine enthält, und bei dem ein Teil des Sauerstoffs in dem Reaktionseinsatz mit einem Teil des oder der Paraffine unter Erhalt eines Prozessgases durch ein oxidatives Verfahren, insbesondere durch eine oxidative Dehydrierung (1) oder eine oxidative Methankopplung, zu dem oder den Olefinen umgesetzt wird, wobei das Prozessgas zumindest den nicht umgesetzten Teil des oder der Paraffine und des Sauerstoffs, das oder die Olefine, ein oder mehrere Acetylene, Kohlendioxid und Wasser enthält, **dadurch gekennzeichnet, dass** das Verfahren umfasst, dass das Prozessgas oder ein Gasgemisch, das unter Verwendung zumindest eines Teils des Prozessgases gebildet wird, teilweise oder vollständig einer zumindest teilweisen Entfernung (4) des Sauerstoffs und des oder der Acetylene unterworfen wird, wobei die zumindest teilweise Entfernung des Sauerstoffs und des oder der Acetylene durch katalytische Umsetzung unter Einsatz eines Kupferoxid oder Ruthenium enthaltenden Katalysators erfolgt, und wobei die katalytische Umsetzung zumindest zum Teil in Form einer Hydrierung erfolgt.

2. Verfahren (100) nach Anspruch 1, bei dem die zumindest teilweise Entfernung (4) des Sauerstoffs und des oder der Acetylene durchgeführt wird, nachdem das Prozessgas oder ein Teil hiervon teilweise oder vollständig einer Kondensatabscheidung (2) und einer Verdichtung (3) unterworfen wurde und bevor ein nach der zumindest teilweisen Entfernung (4) des Sauerstoffs und des oder der Acetylene verbleibendes Gasgemisch zumindest teilweise einer oder mehreren Stufen einer Kohlendioxidentfernung (5) unterworfen wird.

3. Verfahren (100) nach Anspruch 2, bei dem die zumindest teilweise Entfernung (4) des Sauerstoffs und des oder der Acetylene stromab einer oder mehrerer Stufen der Kohlendioxidentfernung (5) und stromauf einer oder mehrerer weiterer Stufen der Kohlendioxidentfernung (5) durchgeführt wird.

4. Verfahren (100) nach Anspruch 2 oder 3, bei dem stromab der zumindest teilweise Entfernung des Sauerstoffs und des oder der Acetylene eine Trocknung (6) und ein oder mehrere Trennschritte (7) durchgeführt werden.

5. Verfahren (100) nach einem der vorstehenden Ansprüche, bei dem der Kupferoxid enthaltende Katalysator eingesetzt wird, und bei dem die zumindest teilweise Entfernung (4) des Sauerstoffs und des oder der Acetylene eine katalytische Hydrierung oder eine zumindest teilweise Umsetzung des Sauerstoffs mit Kohlenmonoxid umfasst.

6. Verfahren (100) nach Anspruch 5, bei dem die zumindest teilweise Entfernung (4) des Sauerstoffs und des oder der Acetylene bei Reaktionsbedingungen durchgeführt wird, die eine Temperatur von 180 bis 360°C, einen Druck von 1 bis 30 bar abs., eine stündliche Gas-Raumgeschwindigkeit von 1.000 bis 15.000 h⁻¹ und ein Verhältnis von Wasserstoff zu Sauerstoff von 0 bis 5 umfassen.

7. Verfahren (100) nach einem der Ansprüche 1 bis 4, bei dem der Ruthenium enthaltende Katalysator eingesetzt wird und, bei dem die zumindest teilweise Entfernung (4) des Sauerstoffs und des oder der Acetylene eine katalytische Hydrierung umfasst.

8. Verfahren (100) nach Anspruch 7, bei dem die zumindest teilweise Entfernung (4) des Sauerstoffs und des oder der Acetylene bei Reaktionsbedingungen durchgeführt wird, die eine Temperatur von 120 bis 360 °C, einen Druck von 1 bis 30 bar abs., eine stündliche Gas-Raumgeschwindigkeit von 1.000 bis 15.000 h-1 und ein Wasserstoff-/Sauerstoff-Verhältnis von 0 bis 5 umfassen.

9. Verfahren (100) nach einem der Ansprüche 5 bis 8, bei dem die zumindest teilweise Entfernung (4) des Sauerstoffs und des oder der Acetylene unter Zugabe von Wasserstoff durchgeführt wird.

10. Verfahren (100) nach einem der Ansprüche 5 bis 7, bei dem die zumindest teilweise Entfernung (4) des Sauerstoffs und des oder der Acetylene isotherm oder mindestens einstufig adiabat durchgeführt wird.

11. Verfahren (100) nach einem der vorstehenden Ansprüche, bei dem die oxidative Dehydrierung als oxidative Dehydrierung von Ethan durchgeführt wird.

12. Anlage zur Herstellung eines oder mehrerer Olefine, die dafür eingerichtet ist, einen Reaktionseinsatz zu bilden, der Sauerstoff und ein oder mehrere Paraffine enthält, und die dafür eingerichtet ist, einen Teil des Sauerstoffs in dem Reaktionseinsatz mit einem Teil des oder der Paraffine unter Erhalt eines Prozessgases durch ein oxidatives Verfahren, insbesonder durch eine oxidative Dehydrierung (1) oder eine oxidative Methankopplung, zu dem oder den Olefinen umzusetzen, wobei das Prozessgas zumindest den nicht umgesetzten Teil des oder der Paraffine und des Sauerstoffs, das oder die Olefine, ein oder mehrere Acetylene, Kohlendioxid und Wasser enthält, **dadurch gekennzeichnet, dass** die Anlage dafür eingerichtet ist, das Prozessgas oder ein Gasgemisch, das unter Verwendung zumindest eines Teils des Prozessgases gebildet wird, teilweise oder vollständig einer zumindest teilweisen Entfernung (4) des Sauerstoffs und des oder der Acetylene zu unterwerfen, wobei für die zumindest teilweise Entfernung des Sauerstoffs und des oder der Acetylene durch die katalytische Umsetzung ein Kupferoxid oder Ruthenium enthaltender Katalysator bereitgestellt ist, der dazu eingerichtet ist, die katalytische Umsetzung zumindest zum Teil in Form einer Hydrierung zu katalysieren.

13. Anlage nach Anspruch 12, die zur Durchführung eines Verfahrens nach einem der Ansprüche 1 bis 9 eingerichtet ist.
